# EUROPÄISCHE PATENTSCHRIFT

(11) **EP 0 688 536 B1**
(45) Veröffentlichungstag und Bekanntmachung des Hinweises auf die Patenterteilung: **02.08.2000**
(21) Anmeldenummer: 94104568.4
(22) Anmeldetag: 23.03.1994
(51) Int. Cl.: A61B 17/32, A61B 18/12

(54) **Multifunktionales Instrument für die Ultraschall-Chirurgie**
Multifunctional instrument for ultrasonic surgery
Instrument multifonctionnel pour la chirurgie à ultra-son

(43) Veröffentlichungstag der Anmeldung: 27.12.1995
(73) Patentinhaber: Erbe Elektromedizin GmbH., D-72072 Tübingen (DE)
(72) Erfinder: Farin, Günter, D-72070 Tübingen (DE); Fischer, Klaus, D-72202 Nagold-Emmingen (DE); Müller, Dieter, D-73117 Wangen (DE)
(74) Vertreter: Meissner, Bolte & Partner

(56) Entgegenhaltungen:
- EP-A- 0 310 431
- EP-A- 0 463 363
- EP-A- 0 547 772
- WO-A-91/13593

## Beschreibung

Die Erfindung betrifft ein multifunktionales Instrument für die Ultraschall-Chirurgie entsprechend den Oberbegriff des Patentanspruchs 1.
Die Ultraschall-Chirurgie ist eine Methode für gewebeselektive Dissektionen. Die Hochfrequenz-Chirurige ist dagegen eine Methode, mit der alle Gewebe (außer Knochen) weitgehend unselektiv geschnitten werden können. Der Ultraschall-Chirurgie fehlt der Hämostaseeffekt. Die Hochfrequenz-Chirurgie kann dagegen sehr gut zur Hämostase angewendet werden. Die kombinierte Anwendung der Ultraschall-Chirurgie und der Hochfrequenz-Chirurgie kann insbesondere bei endoskopischen Operationen die Eigenschaften beider Methoden ergänzen und hierdurch die Operationstechnik verbessern oder gar Anwendungen ermöglichen, die mit der Ultraschall-Chirurgie oder der Hochfrequenz-Chirurgie allein nicht gut oder gar nicht möglich sind.
Die gewebespezifische Selektivität der Dissektion mit Ultraschall resultiert einerseits aus der unterschiedlichen Reißfestigkeit sowie dem relativen Wassergehalt der verschiedenen Gewebe und andererseits aus der Leistungsdichte des Ultraschalls im Gewebe. Gewebe mit geringer Reißfestigkeit und relativ hohem Wassergehalt (z.B. Parenchym) können bereits mit geringer Leistungsdichte des Ultraschalls fragmentiert werden. Gewebe mit hoher Reißfestigkeit und/oder geringem Wassergehalt (z.B. Bindegewebe, Stroma) erfordern eine relativ hohe Leistungsdichte des Ultraschalls. Die Ultraschall-Dissektion eignet sich somit insbesondere zur selektiven, anatomiegerechten Präparation von Gewebestrukturen, z.B. zur Demaskierung von Organen oder Gefäßen von umhüllendem Fettgewebe, zur Fragmentierung oder Aspiration unerwünschter oder pathologischer Gewebe (z.B. Fettgewebe oder Tumore) sowie zur Resektion parenchymatöser Organe (z.B. Leber). Bei Vorhandensein geeigneter Ultraschall-Applikatoren sowie richtiger Applikation und Dosierung der Leistungsdichte des Ultraschalls ist die selektive Dissektion bzw. Fragmentierung und Aspiration von Geweben in fast allen chirugischen Disziplinen anwendbar. Allerdings behindern zwei Probleme die Anwendung der Ultraschall-Dissektion insbesondere bei endoskopischen Operationen. Dies ist einerseits der fehlende Hämostaseeffekt des Ultraschalls insbesondere während der Resektion parenchymatöser Organe und andererseits der unzureichende oder gar fehlende Schneideeffekt des Ultraschalls in binde- und/oder stützgewebehaltigen Gewebestrukturen. Diese beiden bei der Ultraschall-Chirurgie fehlenden Effekte werden dagegen sehr gut mit der Hochfrequenz-Chirurgie erreicht. Eine Vermeidung von Schwierigkeiten der genannten Art ermöglicht deshalb die kombinierte Anwendung der Ultraschall-Chirurgie und der Hochfrequenz-Chirurgie.

Für die kombinierte Anwendung der Ultraschall-Chirurgie und der Hochfrequenz-Chirurgie stehen verschiedene Techniken zur Verfügung.

Die einfachste Technik der kombinierten Anwendung von Ultraschall-Chirurgie und Hochfrequenz-Chirurgie besteht darin, daß der Operateur abwechselnd ein spezielles Instrument für die Ultraschall-Chirurgie und ein anderes spezielles Instrument für die Hochfrequenz-Chirurgie anwendet. Diese Technik hat zwar den Vorteil, daß beide Instrumente optimal für den jeweiligen Verwendungszweck gestaltet sein können aber leider auch den Nachteil, daß der intraoperative Wechsel der Instrumente insbesondere bei endoskopischer Anwendung unbequem und zeitaufwendig ist.

Eine beispielsweise aus US-A-4,931,047 bekannte Technik, bei welcher ein intraoperativer Instrumentenwechsel vermieden wird, besteht darin, die Ultraschall-Chirurgie und die Hochfrequenz-Chirurgie über die selbe Applikatorspitze eines multifunktionalen Instruments anzuwenden. Hierbei kann der Ultraschall und der HF-Strom gleichzeitig oder abwechselnd appliziert werden. Diese Technik kann jedoch insofern problematisch sein, als während der HF-Strom-Koagulation elektrische Lichtbogen bzw. Funken zwischen der Applikatorspitze und dem Gewebe entstehen können, wodurch eine Funkenerosion und damit Beschädigung der Oberfläche der Applikatorspitze verursacht werden kann. Außerdem kann koaguliertes Gewebe an der Oberfläche der Applikatorspitze festkleben und die Ultraschall-Dissektion behindern oder gar verhindern. Ein weiteres Problem dieser Technik besteht darin, daß die Form der für die Ultraschall-Dissektion optimierten Applikatorspitze zum Schneiden mittels HF-Strom nur bedingt geeignet ist.

Es ist Aufgabe der Erfindung, ein multifunktionales Instrument zu entwickeln, welches einerseits für die selektive Dissektion biologischer Gewebe mit Ultraschall und andererseits für die Blutstillung und/oder zum unselektiven Schneiden auch solcher Gewebe geeignet ist, welche mit Ultraschall nicht geschnitten bzw. fragmentiert werden können, mit welchem Instrument Probleme der genannten Art möglichst weitgehend vermieden werden können. Diese Aufgabe wird erfindungsgemäß durch den Gegenstand des Anspruchs 1 gelöst. Vorteilhafte Ausgestaltungen der Erfindung sind Gegenstand der Unteransprüche.

Bei einem derartigen Instrument können ein Applikator für die Ultraschall-Chirurgie und mindestens ein Applikator für die Hochfrequenz-Chirurgie zum Beispiel zum Schneiden und/oder Koagulieren und/oder ein Applikator für die Laser-Chirurgie so am distalen Ende eines kombinierten Instruments angeordnet werden, daß sowohl die Ultraschall-Chirurgie als auch die Hochfrequenz-Chirurgie und/oder Laser-Chirurgie ohne Instrumentenwechsel bequem und zeitsparend angewendet werden können. Derartig kombinierte Instrumente können dem jeweiligen Anwendungszweck entsprechend angepaßt sein. So kann beispielsweise ein multifunktionales Instrument einerseits mit einer für den jeweiligen Anwendungszweck gestalteten Applikatorspitze für die Ultraschall-Dissektion sowie andererseits mit einer monopolaren oder bipolaren Hakensonde, wie sie beispielsweise für die laparoskopische Cholezystektomie verwendet wird, zum Schneiden und/oder Koagulieren bzw. Blutstillen mittels HF-Strom und/oder mit einer Laser-Sonde zum Trennen bzw. Vaporisieren von Gewebestrukturen und/oder zum Koagulieren von Geweben bzw. zum Blutstillen ausgestattet sein. Die Hakensonde kann außerdem in bekannter Weise auch zum stumpfen Präparieren von Geweben angewendet werden.

Derartige multifunktionale Instrumente sollten ergonomisch und sicherheitstechnisch so gestaltet sein, daß jede Funktion möglichst optimal angewendet werden kann. So kann es z.B. zweckmäßig sein, wenn jeweils eine Funktion optimal zur Verfügung steht, welche benutzt werden soll. Ein erfindungsgemäßes Instrument kann dementsprechend so gestaltet sein, daß beispielsweise eine zum stumpfen Präparieren, Schneiden und/oder Koagulieren verwendbare Hakensonde relativ zum distalen Ende bzw. zur Sondenspitze des Ultraschallapplikators derart verschiebbar ist, daß sie gleichen, größeren oder kleineren Abstand zum Gewebe hat als das distale Ende des Ultraschall-Applikators. Auf diese Weise kann, je nach Bedarf, entweder der HF-Strom-Applikator, beispielsweise die Hakensonde, oder die Sondenspitze des Ultraschall-Applikators in optimale Arbeitsstellung gebracht werden. Das distale Ende eines Laser-Applikators, beispielsweise einer Laser-Sonde, kann relativ zum distalen Ende des Ultraschall-Applikators oder des HF-Strom-Applikators so fixiert werden, daß der Fokus des Lasers zum Schneiden oder zum Koagulieren außerhalb des Bereichs des distalen Endes des Ultraschall-Applikators oder des HF-Strom-Applikators liegt, so daß der Ultraschall-Applikator oder der HF-Strom-Applikator während der Laser-Chirurgie als Abstandhalter bzw. Fokussier- oder Defokussierhilfen benutzt werden können. Als HF-Strom-Applikator kann ein erfindungsgemäßes multifunktionales Instrument auch mit einer Argon-Plasma-Koagulations-Sonde ausgestattet sein.

Außerdem können erfindungsgemäße multifunktionale Instrumente zusätzlich mit Einrichtungen zum Saugen und/oder Spülen ausgestattet sein.

In einer vorteilhaften Weiterentwicklung können die erfindungsgemäßen multifunktionalen Instrumente mit einer Einrichtung ausgestattet sein, welche ein automatisches Vorschieben der HF-Strom-Applikatoren und/oder der Laser-Applikatoren in Arbeitsstellung bzw. ein automatisches Zurückziehen der HF-Strom-Applikatoren und/oder Laser-Applikatoren in Ruhestellung, beispielsweise in Abhängigkeit von Aktivierungssignalen für den Schneide- und/oder Koagulationsmodus oder für den Laser- oder Ultraschallmodus und/oder für den Spül- und/oder Saugmodus ermöglicht. Anhand der Zeichnung sollen Ausführungsbeispiele der Erfindung detaillierter beschrieben werden. Es zeigen:
Fig. 1 eine schematische Darstellung des prinzipiellen Aufbaus eines erfindungsgemäßen multifunktionalen Instruments für die Ultraschall-Chirurgie und für die Hochfrequenz-Chirurgie.
Fig. 2 eine schematische Darstellung eines Ausführungsbeispiels des erfindungsgemäßen multifunktionalen Instruments für die Ultraschall-Chirurgie und zum Koagulieren mittels HF-Strom über eine Koagulationselektrode.
Fig. 3 eine schematische Darstellung eines Ausführungsbeispiels des erfindungsgemäßen multifunktionalen Instruments für die Ultraschall-Chirurgie und zum Koagulieren mittels HF-Strom über eine Argon-Plasma-Koagulations-Sonde.
Fig. 4 eine schematische Darstellung eines Ausführungsbeispiels des erfindungsgemäßen multifunktionalen Instruments für die Ultraschall-Chirurgie und zum Schneiden und Koagulieren mittels HF-Strom sowie zum stumpfen Präparieren.
Fig. 5 eine schematische Darstellung eines Ausführungsbeispiels des erfindungsgemäßen multifunktionalen Instruments für die Ultraschall-Chirurgie, welches mit einer Laser-Sonde zum Schneiden oder Koagulieren ausgestattet ist.
Fig. 6 eine schematische Darstellung eines Ausführungsbeispiels eines erfindungsgemäßen multifunktionalen Instruments entsprechend den vorhergehenden Ausführungsbeispielen, welches zusätzlich mit mindestens einer Einrichtung zum automatischen Vorschieben bzw. Zurückziehen der HF-Strom-Applikatoren und/oder des Laser-Applikators ausgestattet ist.
Fig. 7 eine schematische Darstellung der Saug- und Spülfunktionen eines erfindungsgemäßen multifunktionalen Instruments.

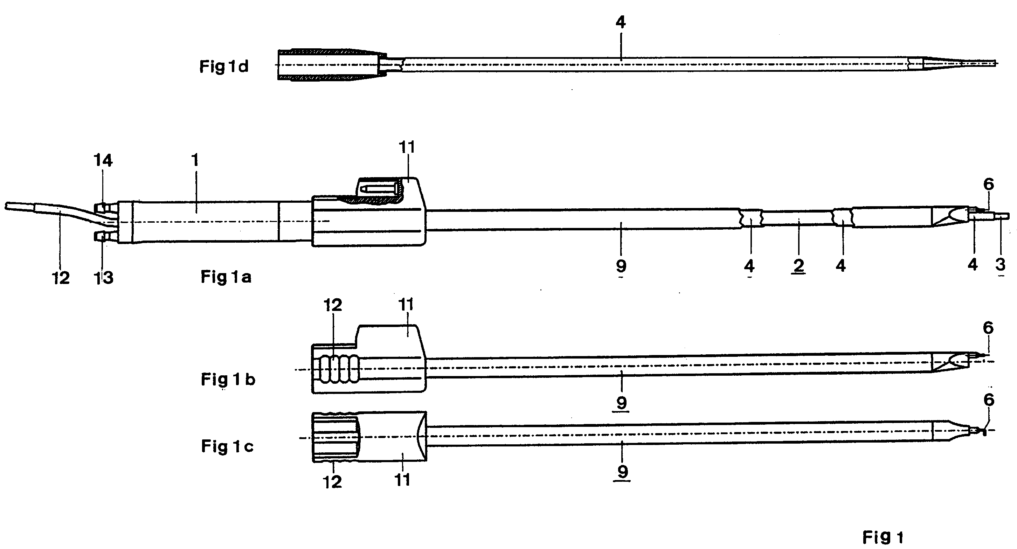

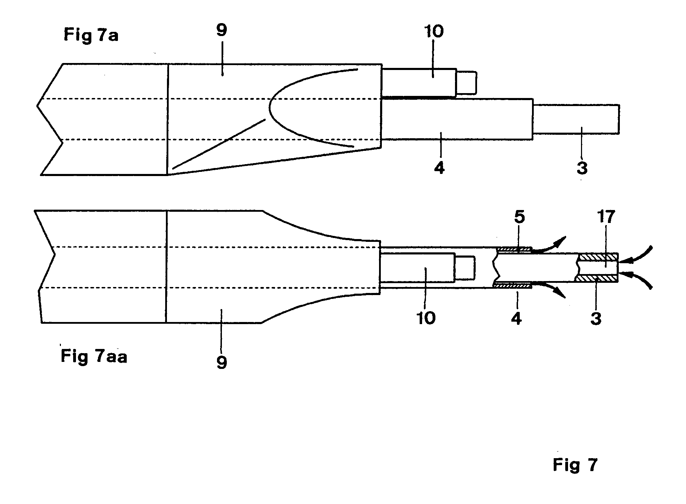

In Fig. 1 ist ein Ausführungsbeispiel eines erfindungsgemäßen multifunktionalen Instruments für die Ultraschall-Chirurgie und für die Hochfrequenz-Chirurgie oder Laser-Chirurgie schematisch dargestellt. Im Griff 1 dieses Instruments ist in an sich bekannter Weise ein elektroakustischer Wandler (nicht sichtbar) vorhanden, an welchen der Ultraschall-Applikator 2 akustisch angekoppelt ist. Der Ultraschall-Applikator besteht in an sich bekannter Weise aus einem Impedanztransformator, dessen distales Ende 3 direkt oder durch Vorschalten eines speziellen Applikator-Tips(nicht dargestellt) mit dem zu fragmentierenden Gewebe in Kontakt gebracht wird. Spezielle Applikator-Tips dienen zur ergonomischen und/oder akustischen Anpassung des distalen Endes des Ultraschall-Applikators an die jeweilige Operationsmethode.
Für Laparoskopische Anwendungen können zwischen elektroakustischem Wandler und Impedanztransformator und/oder zwischen Impedanztransformator und Applikator-Tip in bekannter Weise (Cuschieri) Verlängerungsstücke geschaltet sein (nicht dargestellt), welche den Ultraschall zum distalen Ende 3 des Instruments hin leiten. Bei dem in Fig. 1a dargestellten Ausführungsbeispiel ist der Impedanztransformator inklusive eventuell erforderlicher Verlängerungsstücke stabförmig gestaltet. Um diese stabförmig gestalteten Teile des Instruments kann, wie in Fig. 7 detaillierter dargestellt, koaxial ein Schutzrohr 4 angeordnet sein, dessen Innendurchmesser größer ist als der Außendurchmesser der stabförmigen Teile, welches diese stabförmigen Teile gegen Beschädigung und/oder gegen direkte Berührung schützt. Ein Ausführungsbeispiel eines Schutzrohrs 4 ist in Fig. 1d dargestellt. Der in Fig. 7 dargestellte Spalt 5 zwischen den stabförmigen Teilen und dem Schutzrohr kann in bekannter Weise zum Zuleiten von Spülflüssigkeiten genutzt werden. Über dieses Schutzrohr 4 wird eine Applikatoreinrichtung 9 axial verschiebbar angeordnet, welche an ihrem distalen Ende mindestens einen Applikator 6, beispielsweise eine Elektrode zum Schneiden und/oder Koagulieren mit HF-Strom oder eine Hakenelektrode zum stumpfen Präparieren, Schneiden und/oder Koagulieren oder eine Argon-Plasma-Koagulations-Sonde oder eine Laser-Sonde 15 trägt. Am proximalen Ende der Applikatoreinrichtung 9 ist in diesem Ausführungsbeispiel ein Anschlußteil 11 angeordnet, an welchem die jeweils erforderlichen Medien, wie beispielsweise HF-Strom, Argon und/oder Laser angeschlossen werden. Am proximalen Ende des Griffs 1 des Ultraschallapplikators 2 sind Anschlüsse 12 für den elektroakustischen Wandler 13 zum Saugen und 14 zum Spülen angeordnet. Das Anschlußteil 11 ist ergonomisch so gestaltet, daß es beispielsweise mit dem Daumen der Hand, welche den Griff 1 umschließt und hält, zum axialen Verschieben der Applikatoreinrichtung 9 auf dem Schutzrohr 4 benutzt werden kann. Hierzu kann eine Riffelung 12 die Griffigkeit verbessern.

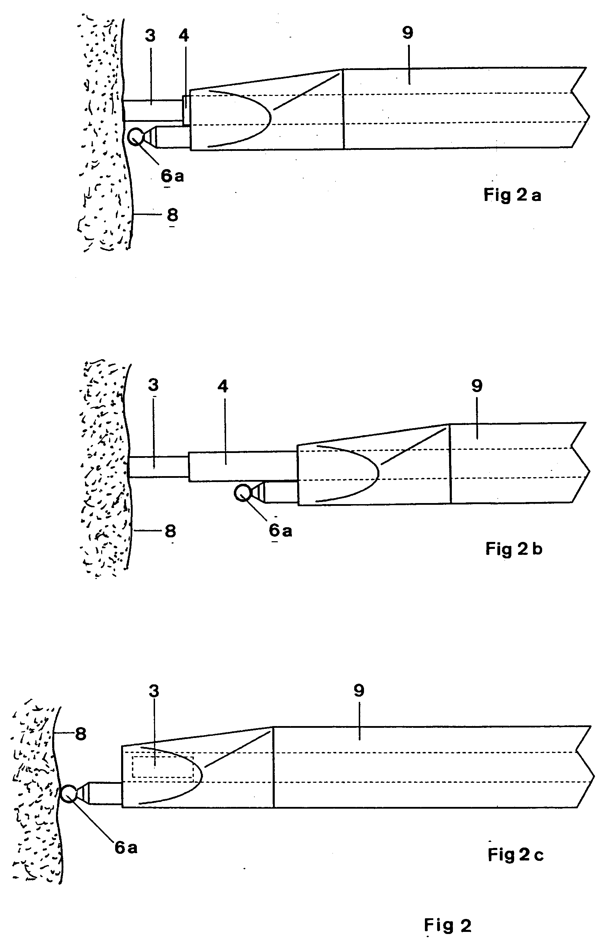

Die Applikatoreinrichtung 9 des in Fig. 1a schematisch dargestellten Ausführungsbeispiels eines erfindungsgemäßen Instruments ist in Fig. 1b und 1c separat aus zwei verschiedenen Perspektiven bzw. Richtungen betrachtet dargestellt. In einer vorteilhaften Ausführung kann die Applikatoreinrichtung 9, wie in Fig. 1 dargestellt, so gestaltet sein, daß sie komplett vom Ultraschallapplikator 2 abnehmbar ist, so daß diese Applikatoreinrichtung 9 bei Bedarf mit dem erfindungsgemäßen multifunktionalen Instrument kombiniert werden kann oder auch nicht. Dies hat außerdem den Vorteil, daß je nach Bedarf eine von verschieden bestückten Applikationseinrichtungen auf den Ultraschallapplikator aufgesteckt und angewendet werden kann. Ausführungsbeispiele verschiedenen Applikationseinrichtungen werden weiter unter dargestellt (Fig. 2,3,4,5) und detaillierter beschrieben.

Die Applikatoreinrichtung besteht vorzugsweise aus elektrisch isolierendem Material. Zur Zuleitung des HF-Stroms vom proximalen Anschlußteil 11 zu den verschiedenen HF-Stromapplikatoren 6 ist eine elektrische Leitung (nicht dargestellt) in dieser Applikatoreinrichtung vorhanden. Für die Verwendung von Argon-Plasma-Koagulations-Sonden oder Laser-Sonden kann die Applikatoreinrichtung mit einem Führungskanal (nicht dargestellt) ausgestattet sein, durch welchen diese Sonden von proximal nach distal eingeführt werden können.

In Fig. 2 ist das distale Ende eines Ausführungsbeispiels eines erfindungsgemäßen multifunktionalen Instruments für die Ultraschall-Chirurgie und für die Hochfrequenz-Chirurgie schematisch dargestellt. Der HF-Strom-Applikator ist in diesem Ausführungsbeispiel eine monopolare kugelförmige Koagulationselektrode 6a. Durch axiales Verschieben der Applikatoreinrichtung 9 auf dem Schutzrohr 4 des Ultraschall-Applikators 2 kann das distale Ende 3 des Ultraschall-Applikators und die Koagulationselektrode 6a, wie in Fig. 2a dargestellt, in gleiche Entfernung zur Gewebeoberfläche 8 oder so positioniert werden, daß das distale Ende 3 des Ultraschall-Applikators , wie in Fig. 2b dargestellt, dem Gewebe 8 näher ist als die Koagulationselektrode 6a,oder die Koagulationselektrode 6a, wie in Fig. 2c dargestellt, dem Gewebe 8 näher ist als das distale Ende des Ultraschall-Applikators 3. Auf diese Weise können Ultraschalldissektion und HF-Koagulation abwechselnd angewendet werden.

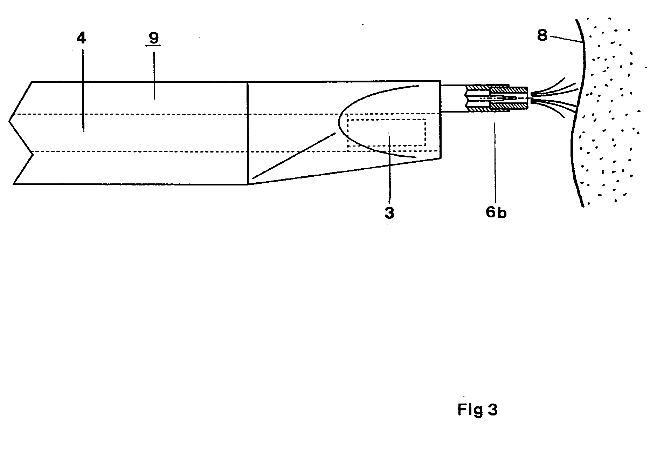

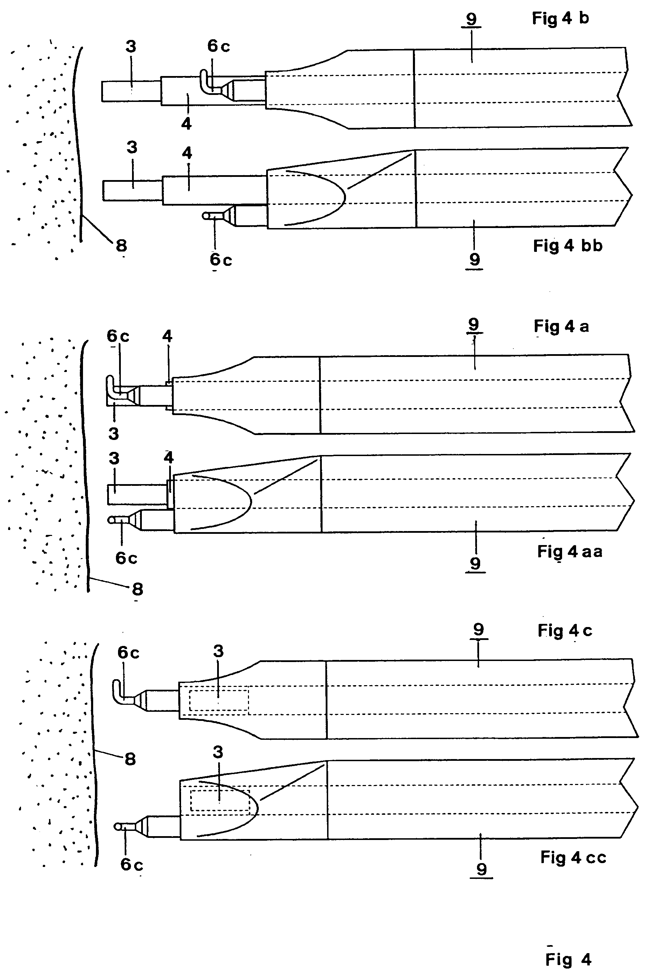

In Fig. 3 ist ein Ausführungsbeispiel eines erfindungsgemäßen multifunktionalen Instruments für die Ultraschall-Chirurgie und für die Hochfrequenz-Chirurgie schematisch dargestellt. Der HF-Strom-Applikator ist in diesem Ausführungsbeispiel eine Argon-Plasma-Koagulations-Sonde 6b. Durch axiales Verschieben der Applikatoreinrichtung 9 auf dem Schutzrohr 4 des Ultraschall-Applikators 2 kann das distale Ende 3 des Ultraschall-Applikators und die Aron-Plasma-Koagulations-Sonde 6b in gleiche Entfernung zur Gewebeoberfläche 8 oder so positioniert werden, daß das distale Ende 3 des Ultraschall-Applikators dem Gewebe 8 näher ist als die Argon-Plasma-Koagulations-Sonde 6b,oder die Argon-Plasma-Koagulations-Sonde 6b dem Gewebe 8 näher ist als das distale Ende des Ultraschall-Applikators 3. Diese Positionierungen sind bei diesem Ausführungsbeispiel nicht zeichnerisch dargestellt, weil sie prinzipiell den in den Fig. 2 sowie 4 entsprechen. Auf diese Weise können Ultraschalldissektion und Argon-Plasma-Koagulation abwechselnd angewendet werden.

In Fig. 4 ist ein Ausführungsbeispiel eines erfindungsgemäßen multifunktionalen Instruments für die Ulraschall-Chirurgie und für die Hochfrequenz-Chirurgie schematisch dargestellt. Der HF-Strom-Applikator ist in diesem Ausführungsbeispiel eine Hakensonde 6c, wie sie beispielsweise für die laparoskopische Cholezystektomie angewendet wird. Durch axiales Verschieben der Applikatoreinrichtung 9 auf dem Schutzrohr 4 des Ultraschall-Applikators 2 kann das distale Ende des Ultraschall-Applikators 3 und die Hakensonde 6c, wie in Fig. 2a bzw. 4a dargestellt, in gleiche Entfernung zur Gewebeoberfläche 8 oder so positioniert werden, daß das distale Ende 3 des Ultraschall-Applikators, wie in Fig. 4b bzw. 4bb dargestellt, dem Gewebe 8 näher ist als die Hakensonde 6c,oder die Hakensonde 6c, wie in Fig. 4c bzw. 4cc dargestellt, dem Gewebe 8 näher ist als das distale Ende des Ultraschall-Applikators 3. Auf diese Weise können Ultraschalldissektion und stumpfes Präparieren oder Schneiden und/oder Koagulieren mit HF-Strom abwechselnd angewendet werden.

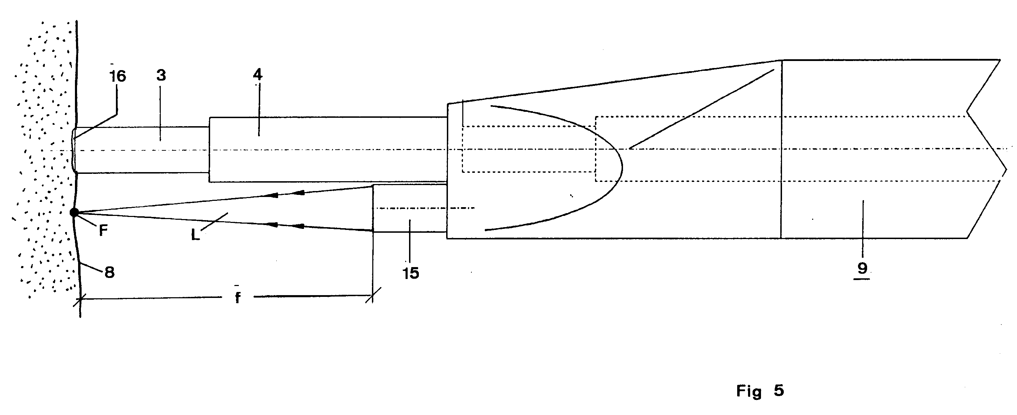

In Fig. 5 ist ein Ausführungsbeispiel eines erfindungsgemäßen multifunktionalen Instruments für die Ultraschall-Chirurgie und für die Laser-Chirurgie schematisch dargestellt. Die Laser-Sonde 15 ist von proximal nach distal durch die Applikatoreinrichtung 9 eingeführt und in dieser Applikatoreinrichtung 9 fixiert. Durch axiales Verschieben der Applikatoreinrichtng 9 nach proximal auf dem Schutzrohr 4 des Ultraschall-Applikators 2 kann die Laser-Sonde 15 in einer derartigen Entfernung f zur Gewebeoberfläche 8 eingerichtet werden, dap der Fokus F des Lasers L in der Ebene der Kontaktfläche 16 des distalen Endes 3 des Ultraschall-Applikators 2 liegt. Auf diese Weise kann das distale Ende 3 des Ultraschall-Applikators 2 als Distanzhalter für die Laserapplikation zum Zwecke der Gewebevaporisation bzw. zum Gewebetrennen angewendet werden. Wird die Applikatoreinrichtung 9 mit der Laser-Sonde 15 aus dieser Position heraus nach proximal oder nach distal auf dem Schutzrohr 4 des Ultraschall-Applikators verschoben, so wird der Laser L auf der Gewebeoberfläche defokussiert und damit die Leistungsdichte reduziert, wodurch der Vaporisationseffekt des Lasers zugunsten des Koagulationseffekts verändert werden kann.

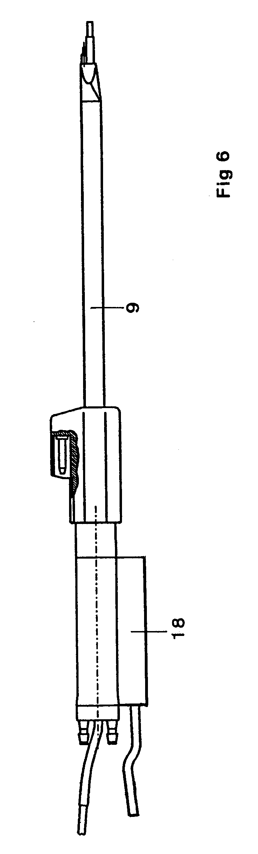

Fig. 6 zeigt eine schematische Darstellung eines Ausführungsbeispiels eines erfindungsgemäßen multifunktionalen Instruments entsprechend den vorhergehenden Ausführungsbeispielen, welches zusätzlich mit einer Einrichtung 18 zum automatischen Vorschieben bzw. Zurückziehen der Applikatoreinrichtung 9 mit dem jeweiligen HF-Strom-Applikator und/oder Laser-Applikator ausgestattet ist. Diese Einrichtung 18 wird im folgenden Aktor genannt. Dieser Aktor kann beispielsweise elektromagnetisch, pneumatisch oder hydraulisch arbeiten. Derartige Aktoren sind an sich bekannt (G. Farin: Pneumatically Controlled Bipolar Cutting Instrument, in Endoscopic Surgery und Allied Technologies, Heft 2, 1993, Seiten 97 bis 101, Verlag Thieme Stuttgart) und deswegen hier nicht detaillierter beschrieben. Das Vorschieben oder Zurückziehen der Applikatoreinrichtung kann automatisch durch die Aktivierungssignale des jeweils an das Instrument angeschlossenen Hochfrequenz-Chirurgiegerät Laser-Gerät oder Ultraschall-Gerät ausgelöst werden. So kann beispielsweise ein Aktivierungssignal "Schneiden mit HF-Strom" den Aktor so steuern, daß eine Hakenelektrode, wie in Fig. 4c dargestellt, automatisch in Arbeitsstellung vorgeschogen wird. Der Aktuator kann beispielsweise am oder im Griff 1 des Instruments angeordnet sein.

Das Ausführungsbeispiel entsprechend Fig. 6 dient zur Erläuterung des Prinzips des automatischen Verschiebens der HF-chirurgischen bzw. der Laser-Applikatoren relativ zum Ultraschall-Applikator. Dieses Verschieben der HF-chirurgischen bzw. der Laser-Applikatoren relativ zum Ultraschall-Applikator kann auch auf andere Weise realisiert werden, beispielsweise derart, daß die Applikatoreinrichtung fester oder austauschbarer Bestandteil des erfindungsgemäßen Instruments ist und die HF-chirurgischen bzw. der Laser-Applikatoren selber manuell oder automatisch durch Aktoren relativ zum Ultraschall-Applikator verschiebbar sind.

Fig. 7 zeigt eine detailliertere Darstellung einer an sich bekannten Saug- und Spülfunktion des Instruments. Bei diesem Ausführungsbeispiel ist neben einem HF-Strom-Applikator 10 an sich beliebiger Art ein Ultraschall-Applikator vorgesehen, der als Rohr ausgebildet ist, dessen Lumen 17 vom distalen Ende 3 bis zum Anschluß 14 zum Saugen als Saugkanal verwendbar ist. Der zum Spülen verwendbare Spalt 5 zwischen dem Ultraschall-Applikator 2 und dem Schutzrohr 4 wurde bereits oben beschrieben.

Bei den dargestellten und beschriebenen Ausführungsbeispielen ist eine Verstellbarkeit der Applikatoren durch eine Verschiebung in Längsrichtung vorgesehen. In gewissen Fällen ist es jedoch vorteilhaft, die Applikatoren in einer Revolvereinheit vorzusehen, die um ihre Längsachse drehbar an dem Instrument angeordnet ist. Wenn beispielsweise drei Applikatoren vorgesehen sind und wenn sich zunächst das distale Ende 3 des Ultraschall-Applikators in der Arbeitsstellung befindet, kann das distale Ende eines anderen Applikators automatisch in die gleiche Arbeitsstellung gebracht werden, indem der erste Applikator zunächst zurückgezogen wird und dann die Revolvereinheit so gedreht wird, daß der zweite oder dritte Applikator nach einem geeigneten Vorschub zur Erzielung eines vorherbestimmten Abstands zu dem Gewebe in eine geeignete Arbeitsstellung gebracht werden kann, wobei das Instrument als solches nicht bewegt werden muß. Wie bei dem erwähnten bekannten pneumatisch gesteuerten multifunktionalen Instrument kann die Steuereinheit bei Ausführungsbeispielen der Erfindung eine logische Schaltung mit einem Mikroprozessor enthalten, dessen Programm die automatischen Einstellungen für die vorgesehenen Funktionen ermöglicht.

## Patentansprüche

1. Multifunktionales Instrument für die Ultraschall-Chirurgie, mit einem Applikator (2) zur Zufuhr von Ultraschall für eine Dissektion von biologischem Gewebe und mit einer Einrichtung zum Schneiden und/oder Koagulieren des Gewebes, **dadurch gekennzeichnet,** daß an dem Instrument mindestens ein von dem Ultraschall-Applikator (2) getrennter HF-Strom-Applikator (6) oder Laser-Applikator (15) in einem seitlichen Abstand vom distalen Ende (3) des Ultraschall-Applikators (2) verstellbar angeordnet ist.

2. Multifunktionales Instrument nach Anspruch 1, **dadurch gekennzeichnet,** daß jeder Applikator (2,6,15) in Längsrichtung verschiebbar in einem gemeinsamen Applikatorgehäuse (9) angeordnet ist.

3. Multifunktionales Instrument nach Anspruch 1 oder 2, **dadurch gekennzeichnet,** daß die Applikatoren (2,6,15) in einer Revolvereinheit vorgesehen sind, die um ihre Längsachse drehbar an dem Instrument angeordnet ist.

4. Multifunktionales Instrument nach einem der vorhergehenden Ansprüche, **dadurch gekennzeichnet,** daß mindestens ein Aktor (18) vorgesehen ist, mit dem ein Applikator (2,6,15) automatisch in eine vorherbestimmte Stellung relativ zum distalen Ende (3) des Ultraschall-Applikators (2) verstellbar ist.

5. Multifunktionales Instrument nach Anspruch 4, **dadurch gekennzeichnet,** daß der Aktor (18) eine elektrisch, pneumatisch oder hydraulisch betätigbare Antriebseinrichtung aufweist.

6. Multifunktionales Instrument nach einem der vorhergehenden Ansprüche, **dadurch gekennzeichnet,** daß der Ultraschall-Applikator (3) gegen den bzw. die HF-Strom-Applikatoren (6) elektrisch isoliert ist.

7. Multifunktionales Instrument nach einem der vorhergehenden Ansprüche, **dadurch gekennzeichnet,** daß der HF-Strom-Applikator (6) als Tasthaken für die Cholezystektomie ausgebildet ist.

8. Multifunktionales Instrument nach Anspruch 1, **dadurch gekennzeichnet,** daß der HF-Strom-Applikator (6) eine monopolare Koagulationselektrode ist.

9. Multifunktionales Instrument nach einem der Ansprüche 1 bis 6, **dadurch gekennzeichnet,** daß der HF-Strom-Applikator (6b) eine Argon-Plasma-Koagulations-Sonde ist.

10. Multifunktionales Instrument nach einem der vorhergehenden Ansprüche, **dadurch gekennzeichnet,** daß ein Saugkanal (17) und/oder ein Spülkanal (5) vorgesehen ist, um mittels des Ultraschalls fragmentiertes Gewebe abzusaugen und/oder Spülflüssigkeit in das Operationsfeld einzuleiten.

## Claims

1. Multifunctional instrument for ultrasound surgery, with an applicator (2) to supply ultrasound for a dissection of biological tissue and with a device for cutting and/or coagulating the tissue, characterized in that there is displaceably disposed at the instrument, separate from the ultrasound applicator (2) and spaced apart laterally from the distal end (3) of the ultrasound applicator (2), at least one HF-current applicator (6) or laser applicator (15).

2. Multifunctional instrument according to Claim 1, characterized in that each applicator (2, 6, 15) is disposed in a common applicator housing (9) so as to be be displaceable in the long direction.

3. Multifunctional instrument according to Claim 1 or 2, characterized in that the applicators (2, 6, 15) are provided in a revolver unit that is so disposed at the instrument as to be rotatable about its long axis.

4. Multifunctional instrument according to one of the preceding claims, characterized in that at least one actuator (18) is provided, with which an applicator (2, 6, 15) can be automatically displaced into a predetermined position relative to the distal end (3) of the ultrasound applicator (2).

5. Multifunctional instrument according to Claim 4, characterized in that the actuator (18) comprises an electrically, pneumatically or hydraulically operated driving device.

6. Multifunctional instrument according to one of the preceding claims, characterized in that the ultrasound applicator (3) is electrically insulated from the HF-current applicator or applicators (6).

7. Multifunctional instrument according to one of the preceding claims, characterized in that the HF-current applicator (6) is constructed as a feeler hook for cholecystectomy.

8. Multifunctional instrument according to Claim 1, characterized in that the HF-current applicator (6) is a monopolar coagulation electrode.

9. Multifunctional instrument according to one of the Claims 1 to 6, characterized in that the HF-current applicator (6b) is an argon-plasma coagulation probe.

10. Multifunctional instrument according to one of the preceding claims, characterized in that a suction channel (17) and/or a rinsing channel (5) is provided for the purpose of sucking away tissue fragmented by the ultrasound and/or introducing a rinsing fluid into the operation field.

## Revendications

1. Instrument multifonctions pour la chirurgie aux ultrasons, avec un applicateur (2) pour l'arrivée d'ultrasons pour une dissection de tissu biologique et avec un dispositif pour inciser et/ou coaguler le tissu, caractérisé en ce qu'au moins un applicateur de courant HF(6) ou un applicateur laser (15), séparé de l'applicateur d'ultrasons (2), est disposé avec une possibilité de réglage sur l'instrument à une distance latérale de l'extrémité distale (3) de l'applicateur d'ultrasons (2).

2. Instrument multifonctions suivant la revendication 1, caractérisé en ce que chaque applicateur (2, 6, 15) est disposé dans un boîtier d'applicateur commun (9) avec une possibilité de déplacement dans le sens longitudinal.

3. Instrument multifonctions suivant l'une des revendications 1 et 2, caractérisé en ce que les applicateurs (2, 6, 15) sont prévus dans une unité revolver, disposée sur l'instrument avec une possibilité de rotation autour de son axe longitudinal.

4. Instrument multifonctions suivant l'une des revendications précédentes, caractérisé en ce qu'au moins un acteur (18) est prévu, par lequel un applicateur (2, 6, 15) peut être réglé automatiquement dans une position prédéfinie par rapport à l'extrémité distale (3) de l'applicateur d'ultrasons (2).

5. Instrument multifonctions suivant la revendication 4, caractérisé en ce que l'acteur (18) présente un dispositif d'entraînement à commande électrique, pneumatique ou hydraulique.

6. Instrument multifonctions suivant l'une des revendications précédentes, caractérisé en ce que l'applicateur d'ultrasons (3) est isolé électriquement par rapport au et/ou aux applicateurs de courant HF (6).

7. Instrument multifonctions suivant l'une des revendications précédentes, caractérisé en ce que l'applicateur de courant HF (6) est réalisé sous forme de crochet de contact pour la cholécystectomie.

8. Instrument multifonctions suivant la revendication 1, caractérisé en ce que l'applicateur de courant HF (6) est une électrode de coagulation unipolaire.

9. Instrument multifonctions suivant l'une des revendications 1 à 6, caractérisé en ce que l'applicateur de courant HF (6b) est une sonde de coagulation du plasma à argon.

10. Instrument multifonctions suivant l'une des revendications précédentes, caractérisé en ce qu'un canal d'aspiration (17) et/ou un canal de lavage (5) est prévu pour aspirer du tissu fragmenté aux ultrasons et/ou pour introduire du liquide de lavage dans le champ opératoire.
